# EUROPEAN PATENT APPLICATION

(11) **EP 3 882 332 A1**
(43) Date of publication of application: **22.09.2021**
(21) Application number: 19883676.9
(22) Date of filing: 06.11.2019
(51) Int. Cl.: C12M 1/42, C12M 3/06

(54) **METHOD AND APPARATUS FOR CONTROLLING INTRACELLULAR DELIVERY OF MATERIALS**

(30) Priority: 12.11.2018 KR 20180138167
(71) Applicant: Femtobiomed Inc., Seongnam-si, Gyeonggi-do 13487 (KR)
(72) Inventor: LEE, Sanghyun, Senongnam-si, Gyeonggi-do 13526 (KR); HA, Sungjae, Senongnam-si, Gyeonggi-do 13602 (KR)
(74) Representative: Botti, Mario
(86) International application number: PCT/KR2019/014988
(87) International publication number: WO 2020/101254

(57) **Abstract**

The present invention relates to: a CellShot platform which is for the injection of a material into a flowing cells and in which a high speed flow cytometer and a 2D injector for intracellular delivery are coupled; and a method for controlling the intracellular delivery of materials by using same. When using a CellShot platform according to the present invention, a material to be delivered can be injected into flowing cells with high throughput and excellent injection efficiency, and macromolecules can be effectively delivered and the survival rate of cells after the injection is excellent, and thus the platform can be widely applied to multiplexed customized cancer vaccine development and advanced cell research.

## Description

### TECHNICAL FIELD

The present disclosure relates to a CellShot platform for the injection of a material into a flowing cell, in which a high-speed flow cytometer is connected to a 2D intracellular delivery injector, and a method of controlling the delivery of a material into a cell by using the same.

### BACKGROUND ART

The direct intracellular delivery of large molecules such as functional proteins, nanoparticles, tissue-lysates, and potentially, microorganisms, intracellular organelles, and large molecules such as exosomes, is one of the key protocols emerging in the fields of cell biology and cell therapeutics. However, most of biochemical carrier-based delivery methods such as viral vectors, plasmids, cationic lipids, and cell-penetrating-peptides are not suitable for the direct delivery of large molecules due to the limited capacity, cell-type dependency, and cargo-specific delivery efficiency. Instead, microfluidic carrier-free delivery methods are more suitable, since they have been conceived to remove the negative effects of the carrier-mediated systems, to improve the intracellular delivery efficiency for large molecules, or to advance the controllability of the delivery.

Currently reported microfluidic carrier-free delivery methods can be classified into two groups, depending on whether or not injection materials are separated from cell media. Most of high-throughput delivery methods incorporate simple one-dimensional (ID) concept, where the materials are mixed with the cell media and delivered by diffusion during the moment of disruption. While 1D methods can achieve high-throughput intracellular delivery of relatively small cargos, slowly diffusing large molecules have little chance to be delivered before the disruption pores reseal, and cytotoxicity and dispersive material loss are the major hurdles.

Meanwhile, the 2D delivery concept, wherein an injection material is separated from cell media, can significantly improve the delivery efficiency of large molecules by allowing full freedom to control delivery parameters through minimization of cytotoxicity and dispersive material loss. *Boukany et al.* have demonstrated the remarkable potential of the 2D intracellular delivery of large molecules into the trapped single cells based on the soft-lithographic nanoinjector system (Boukany, P. E. et al., Nat. Nanotechnol. 6, 747-754 (2011)). However, the low-throughput and no systematic harvesting function of the processed cells due to the dead-end cell-trap design are critical drawbacks against commercialization. *Wu et al.* developed another type of 2D delivery system, the massively parallel photothermal BLAST platform, which performs high-throughput pressure-driven injection into adherent cells after laser induced bubble-poration. However, the BLAST platform is not applicable to flowing cells and the massive parallel processing is less precise and poorly controllable at a single cell level (Wu, Y.-C. et al., Nat. Methods 12, 439―444 (2015)).

The inventors of the present disclosure completed a CellShot platform for the injection of a material into a flowing cell, in which a high-speed direct current (DC) flow cytometer is connected to a 2D intracellular delivery injector, and according to the platform, the conductance change induced by fast flowing single cells may be monitored to detect the presence of single cells, the detected signal may be rapidly transmitted to the injector to thereby automatically inject various large molecules into cells, and then automatically recover the cells into which a material is injected, thereby automatically performing the detection of flowing cells and controlled injection of a material into cells.

### DISCLOSURE

### TECHNICAL PROBLEM

Provided is a method of controlling the delivery of a material into a cell in a device including: a first channel 10 through which the cell is injected; a second channel 20 through which the material is injected; a third channel 30 in which the cell with the material injected thereinto is harvested; a cell transfer channel 50 connecting the first channel 10 to the third channel 30; an injector 60 connecting the second channel 20 to the cell transfer channel 50, and a detector, the method including: (a) detecting the presence of a cell passing through the cell transfer channel 50 by the detector; (b) transmitting a signal for the presence of the cell which is detected by the detector to the injector 60; and (c) injecting a material into the cell passing through the cell transfer channel 50 by the injector 60.

### TECHNICAL SOLUTION

According to an aspect of an embodiment, there is provided a method of controlling the delivery of a material into a cell in a device including: a first channel 10 through which the cell is injected; a second channel 20 through which the material is injected; a third channel 30 in which the cell with the material injected thereinto is harvested; a cell transfer channel 50 connecting the first channel 10 to the third channel 30; an injector 60 connecting the second channel 20 to the cell transfer channel 50, and a detector, the method including: (a) detecting the presence of a cell passing through the cell transfer channel 50 by the detector; (b) transmitting a signal detected by the detector to the injector 60; and (c) injecting a material into the cell passing through the cell transfer channel 50 by the injector 60.

In the present disclosure, the detector may detect the presence of the cell by monitoring a change in current or voltage of each channel. In addition, the detector may be a direct current- or alternating current-based flow cytometer.

In the present disclosure, process (a) may be characterized by detecting 500,000 cells or less per minute.

In the present disclosure, process (b) may be characterized by transmitting to the injector 60, a signal for the detected presence of the cell within 0.001 ms to 10 ms.

In the present disclosure, process (c) may be characterized in that the material is injected into the cell by electroporation or electro-osmotic pressure. In addition, process (c) may be characterized by an injection efficiency of 40% or more (number of cells with a material injected thereinto/number of total cells passing through cell transfer channel x 100) and a cell viability after process (c) of 80% or more (number of viable cells/number of total cells with material injected thereinto x 100).

In the present disclosure, the first channel 10 to the third channel 30 may be radially arranged about the injector 60, central portions of the first channel 10 to the third channel 30 may be bent at predetermined angles, wherein the central portions of the first channel 10 and the third channel 30 are connected to each other via the cell transfer channel 50, and the second channel 20 may be connected to the cell transfer channel 50 via the injector 60.

In addition, the first channel 10 and the third channel 30 may be adjacent to each other or arranged opposite to each other.

In addition, the device may further include a fourth channel 40 through which a material is injected.

According to the present disclosure, one or multiple channels through which a material is injected (material injection channel) may be provided, and two or more material injection channels may be adjacent to each other or arranged opposite to each other. In addition, one or multiple injectors 60 connecting the material injection channel to the cell transfer channel 50 may be provided, and a plurality of injectors 60 may be provided in one material injection channel. In this regard, the injector 60 may be connected to all positions, e.g., a starting portion, a central portion, and a harvesting portion, of the cell transfer channel 50.

In the present disclosure, the injector 60 is a nanochannel connecting the second channel 20, through which a material is injected, to the cell transfer channel 50 and having a sub-micrometer size, and the injector 60 and the detector are connected to electrodes, and thus when a signal is transmitted from the detector, an electric field is generated at the electrodes connected to the injector 60, and consequently, the material is injected into the cell via the second channel 20.

In the present disclosure, the cell transfer channel 50 may be characterized by having a cross-sectional shape that allows a tight contact with the cell therein.

In the present disclosure, the cell transfer channel 50 may be characterized in that an inner diameter of the central portion of the cell transfer channel 50 is smaller than inner diameters of opposite ends thereof, and the cell transfer channel 50 may be characterized by a cross-sectional size that allows a tight contact with the cell therein. Preferably, the cell transfer channel 50 may be characterized by having the same cross-sectional size as the cell.

In the present disclosure, the inner diameter of the central portion of the cell transfer channel 50 may range from 2 µm to 200 µm, and the inner diameters of the opposite ends thereof may range from 5 µm to 500 µm. In addition, the injector 60 may have an inner diameter of 50 nm to 5,000 nm.

The present disclosure also provides a device for controlling the delivery of a material into a cell, the device including: the first channel 10 through which the cell is injected; the second channel 20 through which the material is injected; the third channel 30 in which the cell with the material injected thereinto is harvested; the cell transfer channel 50 connecting the first channel 10 to the third channel 30; a detector configured to detect the presence of the cell; and the injector 60 receiving a signal from the detector and injecting the material into the cell.

In the present disclosure, the first channel 10 to the third channel 30 may be radially arranged about the injector 60, central portions of the first channel 10 to the third channel 30 may be bent at predetermined angles, wherein the central portions of the first channel 10 and the third channel 30 are connected to each other via the cell transfer channel 50, and the central portion of the second channel 20 and the cell transfer channel 50 are connected to each other via the injector 60.

In the present disclosure, the first channel 10 and the third channel 30 may be adjacent to each other or arranged opposite to each other. The cell may be injected via the first channel 10 and pass through the cell transfer channel 50 connected to the bent central portion of the first channel 10, and the material may be injected via the second channel 20, and then may be injected into the cell via the injector 60 positioned at the bent central portion of the second channel 20. Subsequently, the cell with the material injected thereinto exits through the third channel 30 connected to the cell transfer channel 50.

In the present disclosure, the cell transfer channel 50 connects the first channel 10 and the third channel 30 so that the material introduced through the second channel 20 is injected into the cell introduced through the first channel 10 and then the cell with the material injected thereinto is harvested, and is configured such that inner diameters of opposite ends of the cell transfer channel 50 are greater than an inner diameter of the central portion thereof.

In addition, the device may further include the fourth channel 40 through which a material is injected.

DC flow cytometry is an old concept introduced by Coulter in 1956, and thanks to its simple concept, DC-based flow cytometry is still preferred for the implementation of a high throughput system. However, electrochemical complexity and very high electric impedance in microfluidics have been obstacles to adopting high-speed DC flow cytometry due to an insufficient SNR, and thus AC-based flow cytometry has been feasible in many microfluidic fields, instead of the DC-based flow cytometry.

In the present disclosure, a femtosecond laser is a laser with a pulse time width of the femto unit 10⁻¹⁵ equivalent to one millionth of nano, in which beams are formed for a very short period of time, thus causing multi-photon phenomena, which have not been easily seen before, and accordingly, phenomena such as high-energy beams with a short wavelength are caused even with low-energy beams with a long wavelength. In particular, when multi-photon phenomena are caused only in a very local area inside a focus, high-resolution machining and visualization may be realized, and heat is not generated when a material is cut.

To enhance the flow cytometry performance of a CellShot microfluidic chip according to the present disclosure, the microfluidic chip may be processed by fs-laser nanoablation in order to form an actual three-dimensional nanoinjection geometrical structure in a monolithic glass substrate without bonding. However, the present disclosure is not limited only to the monolithic formation.

In the present disclosure, the diameter of each channel may be a size in nanometer units to millimeter units.

The cell which is applicable to the present disclosure may be a prokaryotic cell or a eukaryotic cell.

In the present disclosure, the material to be injected into the cell may be a protein, a peptide, a glycoprotein, a lipoprotein, DNA, RNA, antisense RNA, siRNA, a nucleotide, a ribozyme, a plasmid, a chromosome, a virus, a drug, an organic compound, an inorganic compound, hyaluronic acid, collagen, a cell nucleus, mitochondria, the endoplasmic reticulum, the Golgi body, a lysosome, a ribosome, nanoparticles, or a mixture thereof.

### ADVANTAGEOUS EFFECTS

As is apparent from the foregoing description, when a CellShot platform according to the present disclosure is used, a delivery material can be injected into flowing cells at high throughput and with excellent injection efficiency. In this regard, large molecules can also be efficiently delivered and excellent cell viability is exhibited after injection.

### BRIEF DESCRIPTION OF THE DRAWINGS

FIG. 1 illustrates the concept of a CellShot platform for 2D-linked delivery of a material into a cell(FIG. 1a: a view of a CellShot platform, FIG. 1b: a cell detection plot showing conductance changes, FIG. 1c: an injection plot for material delivery, FIG. 1d: an image of an injector of a flow cytometer, FIG. 1e: the case where the shape of the cell transfer channel 50 is the same as the cross-section of a cell, FIG. If the case where the cell transfer channel 50 is non-circular or formed by bonding).

FIG. 2 is a fluorescence image of an electrical system and a delivery material of a CellShot platform(FIG. 2a: a diagram showing the control of high-speed DC flow cytometry, FIG. 2b: the results of observing real-time resistance peaks, FIGS. 2c and 2d: experimental results of single cell valves, FIGS. 2e, 2f, and 2g: customized LED stroboscope photographing results, FIG. 2h: a graph showing fluorescence intensity according to pulse height, FIG. 2i: a graph showing fluorescence intensity according to pulse width, FIG. 2j: a fluorescence image after an injection pulse).

FIG. 3 illustrates cells passing through a channel structure of a CellShot platform and a real-time cytometry plot(FIG. 3a: a real-time cell detection plot, FIGS. 3b and 3c: the channel structure of the CellShot platform, FIGS. 3d, 3e, and 3f: a process of delivering a material into a cell).

FIG. 4 is an image of a customized femtosecond laser device equipped with a CellShot platform(FIG. 4a: the customized femtosecond laser device, FIG. 4b: a computer-controlled 3-axis piezo-scanning stage with handy connectors connected thereto, FIG. 4c: a CellShot device with a PDMS top layer, FIG. 4d: a CellShot device with a glass top layer, FIG. 4e: a CellShot device having a glass top layer and embedded electrodes).

FIG. 5 illustrates the cell viability of a CellShot platform(FIG 5a: CHO-K1, FIG. 5b: K562, FIG. 5c: NK-92MI).

FIG. 6 illustrates the results of confirming the cell viability after a CellShot platform (FIG. 6a: a control of CHO-K1 cells, FIG. 6b: after the CellShot platform of CHO-K1 cells, FIG. 6c: a control of K562 cells, FIG. 6d: after the CellShot platform of K562 cells).

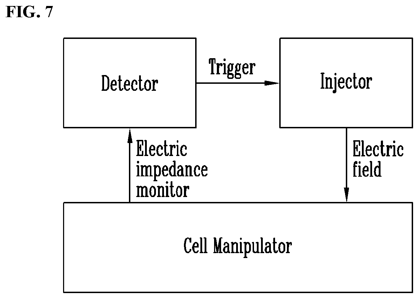

FIG. 7 is a block diagram illustrating the concept of the present disclosure.

### DETAILED DESCRIPTION OF EXEMPLARY EMBODIMENTS

Hereinafter, the present disclosure will be described in further detail with reference to the following examples. It will be obvious to those of ordinary skill in the art that these examples are provided for illustrative purposes only and are not intended to limit the scope of the present disclosure according to the essence of the present disclosure.

### Experimental Setup

A glass-based delivery chip was fixed to a customized connector assembly for fluidic channel loading and electrical connections. The whole assembly was loaded on an inverted microscope (Eclipse Ts2, Nikon, Tokyo, Japan) for visual observation. A delivery material and cells were loaded on the chip by customized pumps. An electric source-meter (2602B, Tektronix Inc., Beaverton, OR, USA) was configured for use as a cell counter and an injection controller. One channel of the source-meter was used in a cytometer and another channel thereof was used to drive an electrokinetic pump. A cell flow channel was filled with a Dulbecco's phosphate-buffered saline (D-PBS, Gibco) solution and electrically connected to be a ground node, and an injection channel was filled with a highly concentrated fluorescein (acid free, Sigma Aldrich) solution and electrically connected to be a source node. Electrical pulse generation at the source was controlled in terms of a pulse width, a current clamping level, and a voltage clamping level. Parameters for a system controller and data monitoring were set in customized PC software.

### Chip Fabrication

A microfluidic chip device was designed at Micronit GmbH (Dortmund, Germany) using borosilicate glass with platinum electrodes. The size of a basic glass electrode chip for a CellShot platform was 22.5 mm × 22.5 mm × 1.2 mm and top and bottom layers thereof were connected. The top layer includes microfluidic channels having a relatively large feature size and a depth of 40 µm, and the bottom layer includes 180 nm-thick electrodes made of platinum on a 10 nm-thick tantalum adhesive layer. A core structure, a cell-processing tunnel, and an injection channel with a nanoinjector were subjected to laser machining in the bottom glass to have monolithic geometric shapes. The size of the nanoinjector may also be adjusted depending on gene-editing materials. A femtosecond laser (fs-laser; Light Conversion Ltd., Vilnius, Lithuania) machining process using a computer-controlled 3-axis piezo-scanning stage (P545.3R7 XYZ nano-stage, Physik Instruments GmbH & Co.) was used to create critical 3-dimensional shapes inside and outside of a transparent material. For the best productivity, a microfluidic chip with a relatively large structure was manufactured by general lithography.

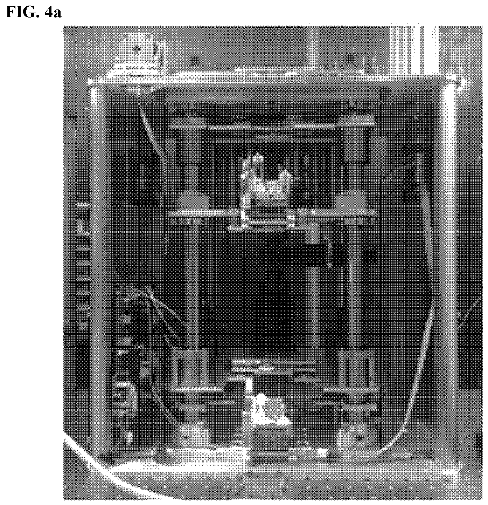

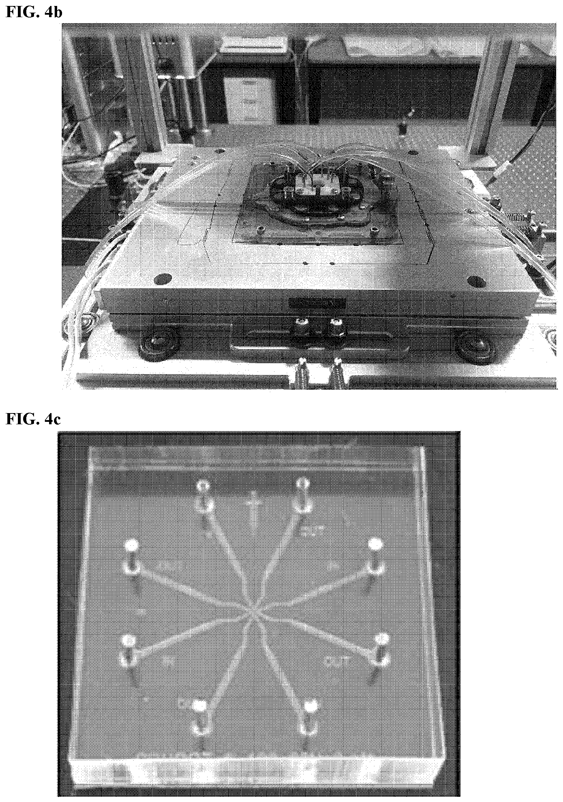

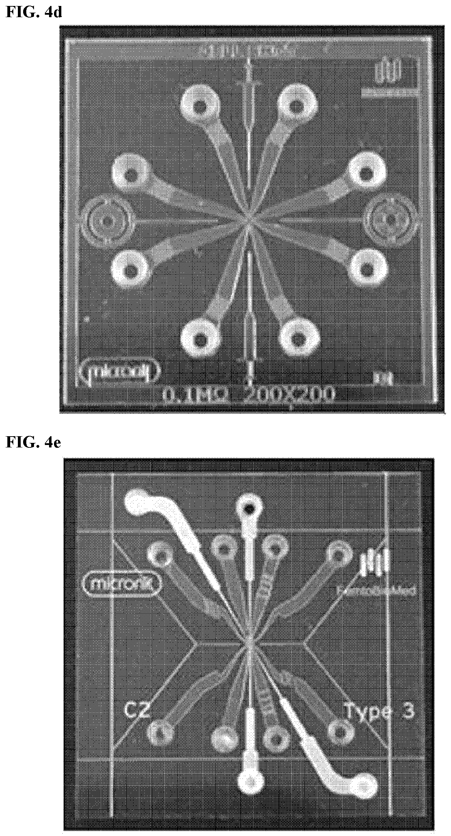

The structures of a customized device for femtosecond laser machining, a computer-controlled 3-axis piezo-scanning stage with handy connectors, a connector assembly for the one-touch closure of a CellShot chip, and a top unit thereof are illustrated in FIG. 4a. For femtosecond laser (fs-laser) nanoablation, an actual 3D nanoinjection structure was engraved at the center of a chip inside a monolithic substrate. The delivery experiments were performed using an inverted microscope with an appropriate electrical connection. As illustrated in FIGS. 4c to 4e, there are three CellShot prototype devices manufactured using a PDMS top layer (see FIG. 4c), a glass top layer (see FIG. 4d), and a glass top layer and embedded electrodes (see FIG. 4e). The core shape was formed inside a lower glass layer regardless of top layer materials. Glass chips were preferred for anti-bacterial treatment and flow control. Metallic electrodes, embedded by an atomic layer deposition process, help reduce electric resistance from a cell-processing tunnel to a current probe and slightly improve a signal-to-noise ratio, but high manufacturing costs and possible bubble generation near the core region at a large bias voltage were drawbacks. Full glass CellShot microfluidic chips without embedded electrodes were preferred in terms of overall performance and production costs.

### Connector Design

A customized connector assembly was designed by connecting general fluidic components to small microfluidic chips and completing an electrical circuit setup with the microfluidic device. The connector assembly was made of commercially available polyetherimide (Ultem®PEI), polytetrafluoroethylene (PTFE), polyether ether ketone (PEEK), polycarbonate (PC), photoreactive resins, and 6060 aluminum, and was fabricated by 3D printing (Form 3, Formlabs / Ultimaker 5S, Ultimaker BV / Intamsys HT, Intamsys Technology Co. Ltd. / Wanhao Duplicator D8, Wanhao) or CNC machining (TinyCNC-6060, Tinyrobo). Several button magnets and a guide pin completed the one-touch closure of the assembly. A chip layout and an assembly part layout were achieved with the Solidworks CAD software (Dassault Systèmes, Vélizy-Villacoublay, France). The 3-axis machining toolpaths for femtosecond laser machining and CNC machining were generated by the VCarve Pro CAD software (Vectric Ltd., Redditch, UK) or Fusion 360 CAD software (Autodesk Inc., CA, USA).

### Cell Culture and Preparation

CHO-K1 cells, which are Chinese hamster ovary cells, were maintained in F-12K media (Gibco) supplemented with 10% (v/v) heat-inactivated FBS (Gibco) and 1% penicillin/streptomycin (Pen/Strep(Gibco)) in humidified air with 5% (v/v) CO₂ at 37 °C. A subcultivation ratio was 1:8. Cell cultured concentration was 2-3×10⁵ cells/ml. Cells were re-suspended at a density of 2×10⁶ cells/ml in 0.22 µm-filtered D-PBS buffer before use (Millipore, Bedford, MA, USA), and the media containing cells were filtered (10 µm) before use.

Meanwhile, K562 cells, which are from a human leukemic cell line, was maintained in RPMI 1640 media (Gibco) supplemented with 10% (v/v) heat-inactivated FBS (Gibco) and 1% Pen/Strep (Gibco) in humidified air with 5% (v/v) CO₂ at 37 °C. Cell culture concentration was 1×10⁵ cells/ml. NK-92MI, which is an interleukin-2 (IL-2) independent natural killer cell line, was free of ribonucleosides and deoxyribonucleosides, but was maintained in an alpha minimum essential medium supplemented with 2 mL of L-glutamine and 1.5 g/L of sodium bicarbonate (Gibco). For the production of a complete growth medium, to a basal medium were added the following components: 0.2 mM inositol (Sigma); 0.1 mM 2-mercaptoethanol (Sigma); 0.02 mM folic acid (Sigma); horse serum (Gibco) to a final concentration of 12.5%; FBS (Gibco) to a final concentration of 12.5% and 1% Pen/Strep in humidified air with 5% (v/v) CO₂ at 37 °C. The cell culture concentration was 2-3×10⁵ cells/ml.

### Delivery Materials

TRITC-conjugated dextran (4.4 kDa, Sigma) was used at 50 mg/ml in D-PBS buffer. A fluorescein solution (saturated free acid, prod. no. 46955, Sigma) in D-PBS buffer was used. All solutions were filtered (0.22 µm) before use to remove undesired particles and a clogging effect.

### Delivery Efficiency and Cell Viability

Cells with a cargo contained inside thereof were counted as cells into which a material had been delivered. From snapshots captured using a fluorescence microscope, injection efficiency was calculated as a ratio of the number of successful cases to a total number of passed cells, and cell viability before/after cells were treated in the CellShot platform was examined. After electrical stimulation, harvested cells were stained with calcein-AM, which is used for labeling live cells. The samples were cultured with 2 µM calcein-AM at room temperature under a light-shielding condition for 20 minutes to 30 minutes. Phase contrast and fluorescence images of cells were acquired for image processing.

### Image Anaylsis

Bright field and fluorescence images were recorded using an inverted microscope by a CMOS camera (PCO.edge, PCO GmbH, Kelheim, Germany). Videos were captured at 60 frames per second with 5 ms exposure time. The images were analyzed in ImageJ image processing software (Version 1.52a, https://imagej.nih.gov), and background noise was excluded from quantitative image analysis.

### CellShot Platform Including Flow Cytometer and Automatic Nanoinjector

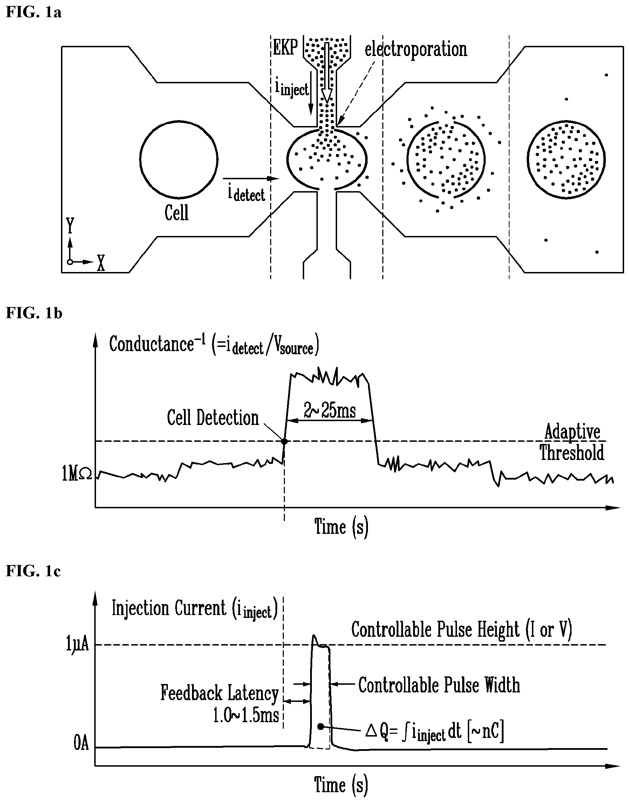

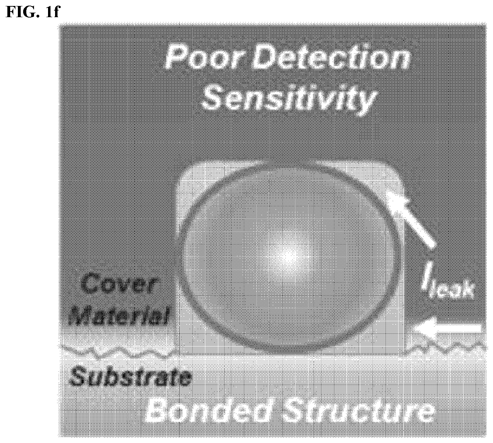

A feature of the CellShot platform is automatic and simultaneously direct intracellular delivery into fast flowing single cells (see FIG. 1a). A high-speed DC flow cytometer for detecting the presence of single cells is connected to a 3D nanoinjector, and the 3D nanoinjector is automatically triggered to apply an electric field along an injection channel by receiving a feedback signal from the flow cytometer within 2 ms. It is not easy to rapidly detect single cells in units of millisecond in conventional lithographic microfluidics due to a poor signal-to-noise ratio (SNR<1). However, the actual 3D geometrical structure of the channel structure and advanced logic algorithms such as adaptive threshold tracking (ATT) of the flow cytometer improved the SNR of cell measurement up to 20.

First, the signal strength of a conventional lithographic rectangular microchannel is usually very weak, on the order of pico to nanoamperes, and it is also difficult for modern electronics to provide a sufficient SNR without the use of time-consuming alternating current (AC) as in lockin-amplification. According to a conductance model assuming the case where electrolyte flow is completely blocked by the presence of a cell, a conductance change may be at a microampere level. However, in existing microfluidic devices, unwanted current leakage generated at the non-circular contact interface is likely to occur, which considerably weakens signal intensity. In addition, incomplete bonding interfaces also cause parasitic current leakage and conductance path breakdown (see FIG. If). In contrast, 3D channels in a monolithic glass substrate, which are formed by a femtosecond laser nanoablation process according to the present disclosure, are able to remove unwanted current leakage and accordingly, enhance signal intensity up to million times.

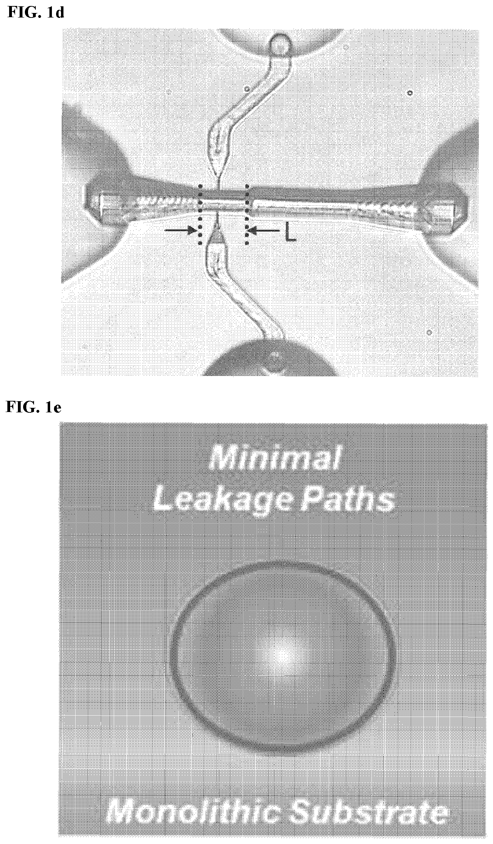

FIG. 1a illustrates the concept of intracellular delivery of a CellShot platform according to the present disclosure, A cell detection plot(FIG. 1b) showing conductance changes, and an injection plot(FIG. 1c) for the delivery of a material induced from a cell detection signal. FIGS. 1d to 1f illustrate that the structure of an injector and a monolithic configuration, according to the present disclosure, can minimize current leakage in an actual 3D image. FIG. 1d shows an image of an injector portion where a cell passing through a channel of a body of a flow cytometer is present, wherein L denotes the length of an injection chamber or the length of the body of a flow cytometer (generally ranging from 12 µm to 36 µm), D1 denotes the diameter of the narrowest portion (generally ranging from 8 µm to 12 µm), and D2 denotes the diameter of a nanoinjector (generally ranging from 400 nm to 1,000 nm). As illustrated in FIG. 1d, an asymmetric channel structure may be applied. FIG. 1e illustrates that current leakage is able to be minimized in the case where the shape of a channel through which a cell passes is almost the same as the cross-section of the cell, and FIG. If illustrates the occurrence of current leakage when the shape of the channel through which a cell passes is non-circular.

Second, in flow cytometry, the ATT algorithm minimizes the effect by noise and the drift of a basic signal due to electrolyte flow. In this regard, diverse types of noise and drift were induced from the capacitive coupling, electrochemical reactions, electrokinetic separation of ions, and abrupt onset of the repulsive ion barrier in an injection nanochannel.

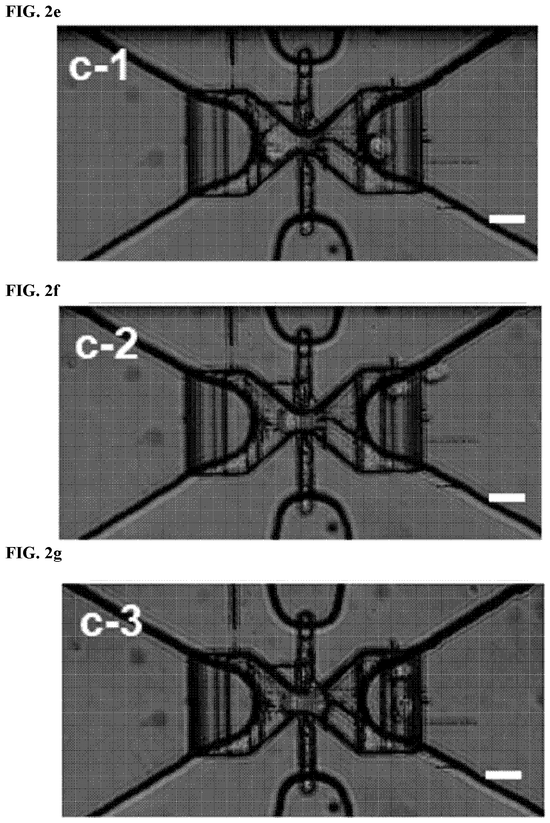

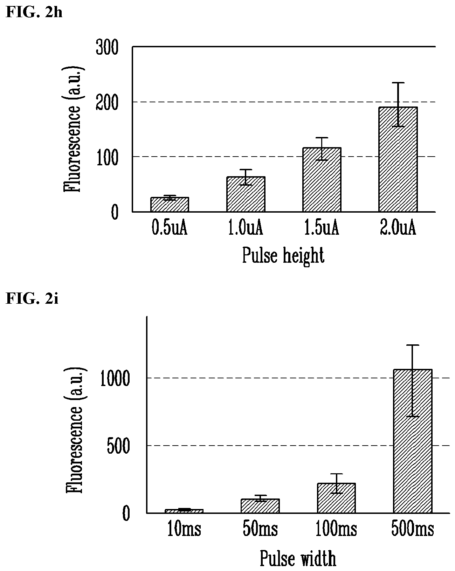

Electric potential automatically triggered along the injection channel is first concentrated on the cell membrane and immediately cause electroporation. Thereafter, the electric potential is distributed through the injection nanochannel, a high electroosmotic pressure (Δ*P∝*1/*D*⁴) is formed at the nanochannel instead of electrophoresis, and accordingly, interfacial electrokinetic pumping (EKP) is mainly induced (see FIGS. 2h to 2j). Provided that electric current is a direct matrix for the actual flow of electrolytes, real-time electric current clamping is applied to control an EKP driving pressure and a flow rate during injection.

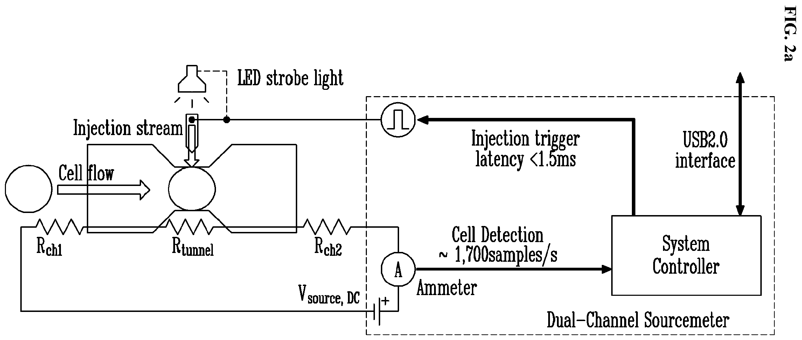

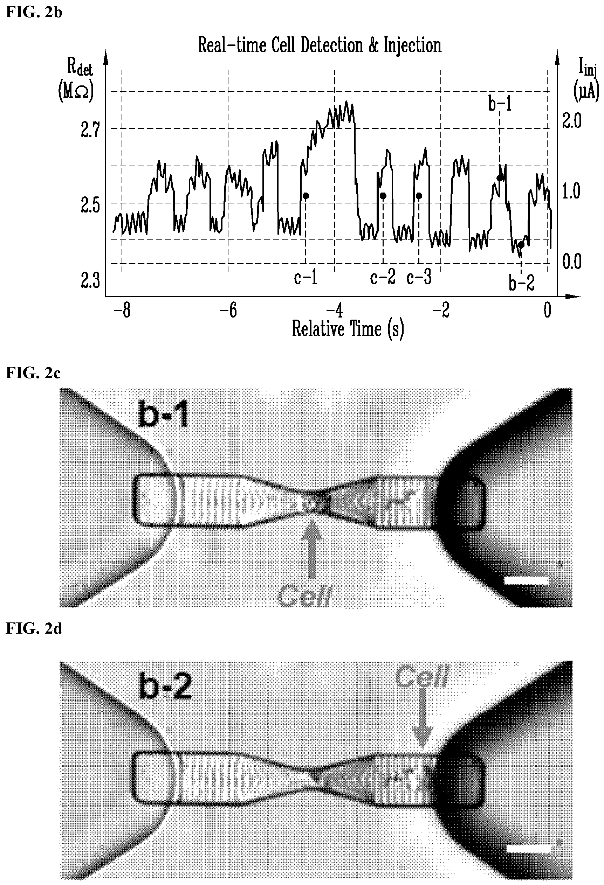

Meanwhile, a diagram showing the control of high-speed DC flow cytometry is schematically illustrated in FIG. 2a. A dual-channel source-meter detects cell flow in a single channel (increase in Rₜᵤₙₙₑₗ), and an injection stream is induced in another channel. As illustrated in FIG. 2b, the optimized 3D structure of a CellShot nanoinjector may usually induce an MΩ resistance peak, which is 10 times greater than the noise of a basic signal. Under an electric field induced by a DC voltage source (V_{source}, DC), ions drift towards electrodes and thus generate electric current, and electric current is measured from electrolyte transfer which varies depending on the DC resistance (R_{ch1} + Rtunnei + R_{ch2}) of the system.

As illustrated in experiments for a single cell valve of a slowly shrinking cell tunnel (see FIGS. 2c and 2d), an accurate resistance peak appeared only when a cell firmly blocked the channel, from which it was confirmed that unwanted current leakage could be removed through the optimized 3D geometrical structure of the DC flow cytometer. The behavior of the resistance peak represents 0 or 1 similar to a logic comparator, which indicates that the flow cytometer is accurate and reliable. In addition, the size and travel time of a cell along with the position thereof may be measured by monitoring a change in electric resistance.

Meanwhile, the central portion of the flow cytometer is an embedded processor of a dual channel source-meter which runs Lua®scripts for cell detection and material injection. The embedded processor not only monitors resistance peaks, but also manages a reference signal according to the ATT algorithm to accurately detect the presence of a cell and trigger injection. During processing, two major latency periods of detection and feedback exist, which are respectively minimized below 1 ms. Thus, the total 2 ms latency indicates a throughput bandwidth of a maximum of 1.8 M cells/h. Another function which does not affect system latency like parameter setting and data monitoring is managed by custom software of an external desktop PC which communicates with a source-meter via a USB interface. To verify the developed high-speed DC flow cytometer, timely exposed photographs were captured using a customized LED stroboscope. As illustrated in FIGS. 2e to 2g, the LED strobe is synchronized with an 2 ms injection pulse and thus is capable of capturing the moment of injection up to 4 ms after detecting the presence of a cell, well proving the smooth operation of the flow cytometer.

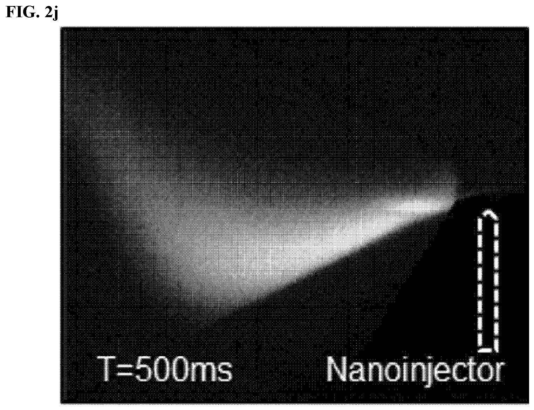

The electrokinetics of an fs-laser-machined nanoinejctor is known to be dominantly electroosmotic pumping, which is less sensitive to the surface charge and molecular weight of molecules compared to electrophoresis. As illustrated in FIGS. 2h to 2j, negatively charged fluorescein molecules were strongly injected from a glass nanoinjector, and the injection behavior was observed to be similar to the injection of large neutral molecules such as a tetramethylrhodamine isothiocyanate (TRITC)-conjugated dextran red fluorescent dye used for intracellular delivery. This is due to highly increased electroosmotic pressure as the overall size of channel diameter is reduced to a sub-micron scale (0.4 µm to 1 µm), whereas electrophoresis is as important as electroosmosis in existing microchannels, wherein only cations are able to pass through a glass nanochannel smaller than 100 nm due to strong ionic rejection. Moreover, the amount of injection was linearly controlled according to electric field intensity and duration, allowing quantitative intracellular delivery with consistency. FIG. 2h illustrates fluorescence intensity according to pulse height, and FIG. 2i illustrates fluorescence intensity according to pulse width. As illustrated in FIG. 2j, fluorescence was calculated by a net pixel value obtained from the background-compensated microscope image after an injection pulse, and a reference background image was selected from frames immediately before the injection pulse was applied.

### Intracellular Delivery of Material Using CellShot Platform

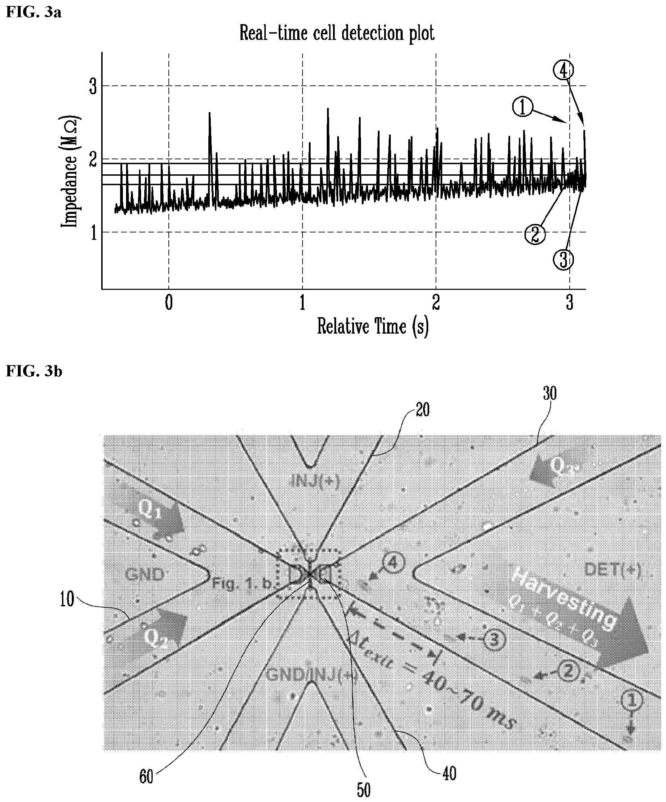

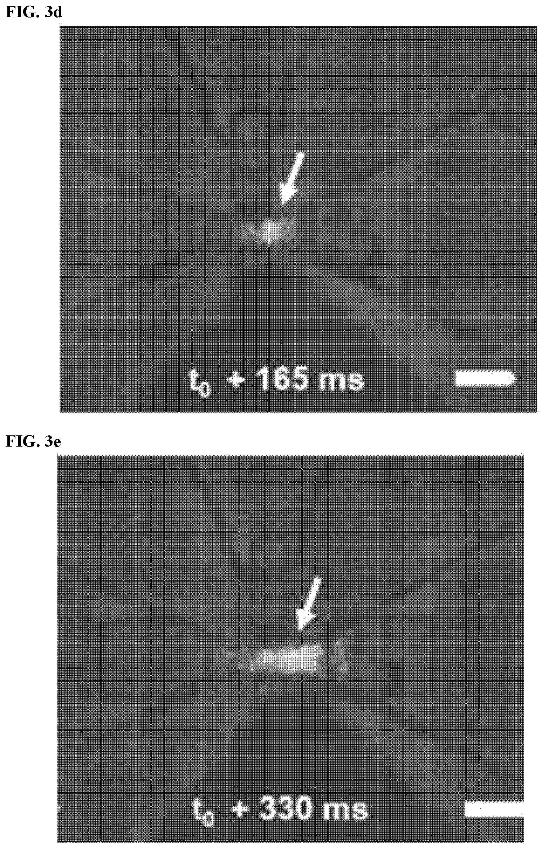

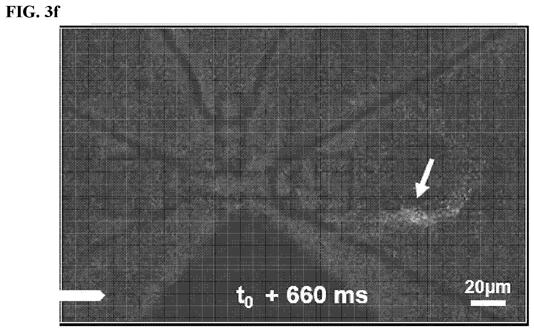

FIG. 3a illustrates impedance peaks by a nanoinejctor as cells pass, as a part of a real-time cell detection plot. The drift of a baseline is often as important as peaks in a noisy environment, but the ATT algorithm can very stably track the drift over time of a signal of the baseline, achieving consistent detection with an almost high SNR at all times. Detection peaks with SNR > 5 were about 1,200 per minute, which is the same as the throughput of~72 K cells/h. Four treated cells shown in FIGS. 3a and 3b are spaced at a time interval of 40 ms to 70 ms (corresponding to 50-90 K cells/h), which was highly consistent with the average throughput of 850 to 1,500 cells per minute.

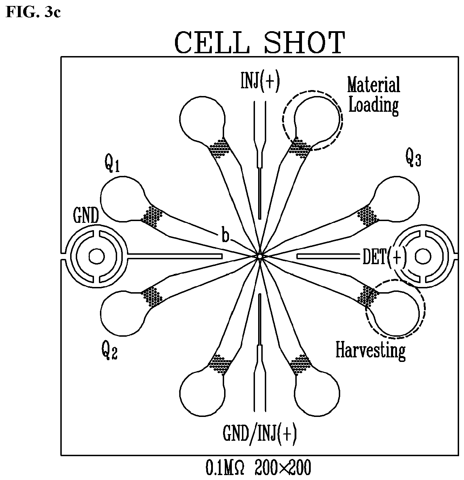

A full glass CellShot chip (see FIG. 3c) actually has 8 inlet ports, wherein a pair of inlet ports constitutes one V-shaped channel to remove air from channels and fill cells with media and an injection material. As illustrated in FIG. 3c, cells enter via the cell inlet ports, the injection material is injected into the cells at the central portion by a nanoinjector, and then transferred to a harvesting chamber. Meanwhile, since manual handling of an 8-port fluidic chip with an electrode at each port is not simple even for skilled operators, one-click connectors were developed to connect a CellShot chip to an electronic device and a fluid control system (see FIG. 4b). A customized femtosecond laser device and a structure thereof are illustrated in FIG. 4 (FIG. 4a: a customized femtosecond laser machining device, FIG. 4b: a computer-controlled 3-axis piezo-scanning stage to which handy connectors are connected, FIG. 4c: a CellShot device with a PDMS top layer, FIG. 4d: a CellShot device with a glass top layer, and FIG. 4e: a CellShot device with a glass top layer and embedded electrodes).

TRITC-dextran dissolved in a D-PBS buffer was delivered into CHO-K1 cells. TRITC-dextran is appropriate as non-membrane-permeable large molecules, and red fluorescence can be easily distinguishable from green fluorescence of Calcein-AM. The detailed processes of the intracellular delivery of the highly concentrated (50 mg/mL) TRITC-dextran solution into CHO-K1 are illustrated (see FIGS. 3d to 3f). Automated injection synchronized with the presence of a cell was smoothly performed, and excessively injected TRITC-dextran by 20 ms-long injection mostly surrounds the CHO-K1 cells even after the cell completely escaped from an injection sector. This further promotes delivery to the outside of the injection sector via diffusive transfer until pores are closed.

Various specific experiments were conducted to finely tune the CellShot platform, and as a result, the delivery efficiency of the CellShot platform was as high as 90.2% when TRITC-dextran was delivered into CHO-K1. Also, most of the cells (~96.4%) survived after high-throughput delivery. Compared with bulk processing such as electroporation and cell-squeezing, injection via nanochannels causes local membrane disruption around contact spots, thus helping maintain the survival of cells. In addition, compared with mechanical disruption methods, CellShot is characterized by minimizing damage to the cytoskeleton and the nucleus by applying uniform and gentle pressure to the cell membrane.

### Delivery Efficiency of Material into Cells

Intracellular delivery experiments were conducted on three different cell types: CHO-K1, K562, and NK92-MI using TRITC-dextran as an injection material. Under a fluorescence microscope (Eclipse Ts2, Nikon, Tokyo, Japan), cell flow and the motion of a fluorescent material were monitored using a highly sensitive image sensor (PCO.edge, PCO GmbH, Kelheim, Germany) at 60 frames per second with 5 ms exposure time. Image processing for distinguishing injection success or failure by intensity calculation was done using imageJ software. From snapshots, a difference in fluorescence intensity between the time points at which TRITC-dextran was successfully or not successfully injected into each cell was confirmed. After background noise was removed, injection efficiency was calculated as a ratio of the number of success cases to the total number of passed cells. In case of CHO-K1, the delivery efficiency was as high as 90.2%, since 74 cells were successfully treated among 82 cells. The K562 and NK92-MI cases resulted in 68.0% (83 out of 122) and 44.2% (19 out of 43), respectively.

For cell viability experiments, the cells were filtered via a 0.22 µm filter in a D-PBS buffer before use and re-suspended at a density of 1-2×10⁶ cells/mL. After electrical stimulation, harvested cells were stained with calcein-AM, which is used for labeling live cells. The samples were cultured with 2 µM calcein-AM at room temperature under a light-shielding condition for 20 minutes to 30 minutes. Phase contrast and fluorescence images of the cells were acquired for image processing. To calculate cell viability, 15 regions were randomly selected from images of each group and cell viability was calculated using the following equation: cell viability (%) = (G/T) × 100%, wherein G denotes the number of cells with a green signal, and T denotes the total number of detected cells.

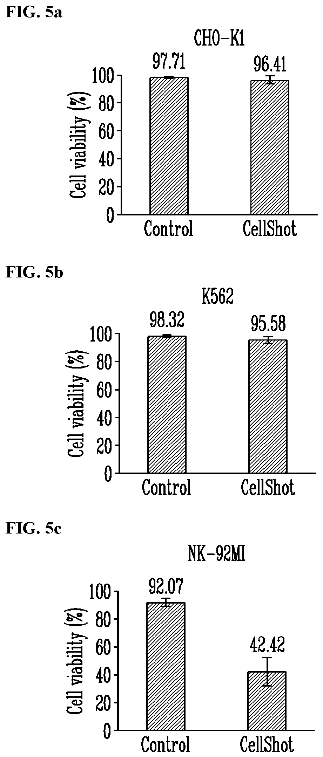

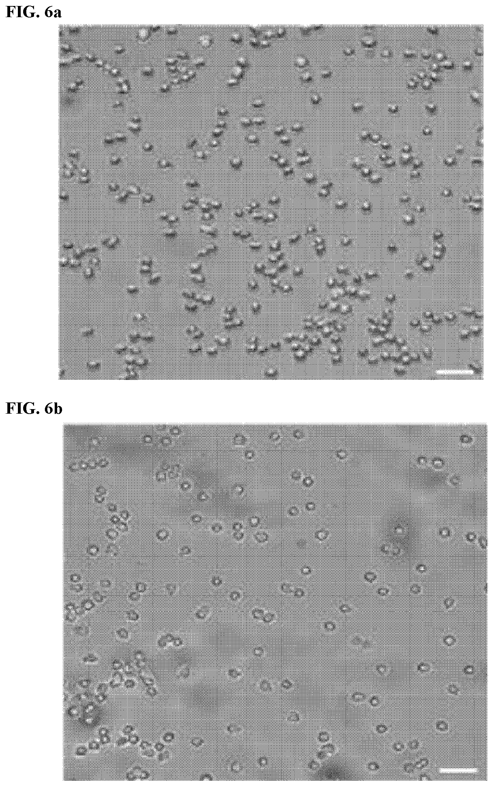

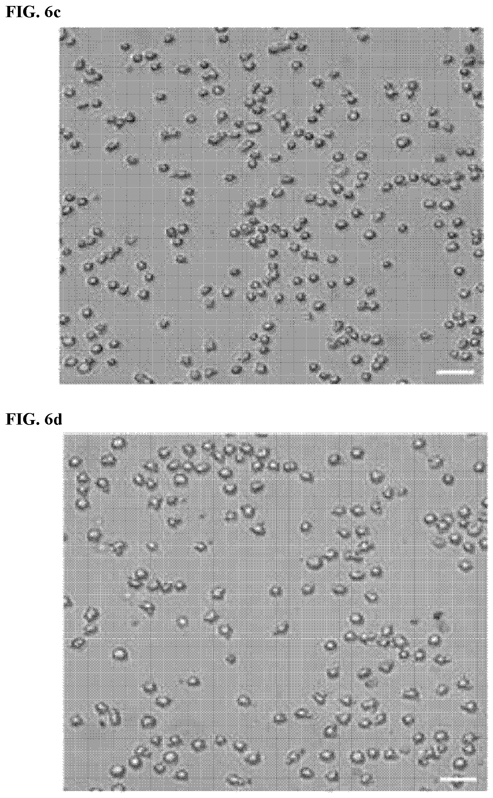

Meanwhile, in cells having a high nucleus-cytoplasm ratio (N:C ratio), such as natural killer (NK) cells, mechanical approaches such as cell-squeezing cause severe damage due to nuclear damage and cytoskeletal collapse. As illustrated in FIGS. 5a to 5c, CHO-K1 and K562 cells exhibited a cell viability of 95% or higher, whereas, a decreased cell viability, i.e., 50% or less, was observed in NK-92MI cells having a very high N:C ratio (-150%) compared to cells having a low N:C ratio (see FIGS. 5a to 5c and 6a to 6d). Meanwhile, microscopic observation results showed that the N:C ratios of respective cell types of CHO-K1, K562, and NK92-MI were 0.3:1, 0.8:1, and 1.5:1, respectively.

## Claims

1. A method of controlling delivery of a material into a cell in a device comprising: a first channel through which the cell is injected; a second channel through which the material is injected; a third channel in which the cell with the material injected therein is harvested; a cell transfer channel connecting the first channel to the third channel; an injector connecting the second channel to the cell transfer channel; and a detector, the method comprising following steps:
(a) detecting, with the detector, a presence of a cell passing through the cell transfer channel;
(b) transmitting a signal detected by the detector to the injector; and
(c) injecting, with the injector, the material into the cell passing through the cell transfer channel.

2. The method of claim 1, wherein in the detecting, the detector detects the presence of the cell by monitoring a change in current or voltage of each channel.

3. The method of claim 1, wherein the detector is a direct current- or alternating current-based flow cytometer.

4. The method of claim 1, wherein the step (a) comprises detecting 500,000 cells or less per minute.

5. The method of claim 1, wherein the step (b) comprises transmitting the signal to the injector within 0.001 ms to 10 ms from the detecting the presence of the cell.

6. The method of claim 1, wherein the step (c) comprises injecting the material into the cell by electroporation or electroosmotic pressure.

7. The method of claim 1, wherein the step (c), injection efficiency is 40% or more, and wherein the injection efficiency is defined as a number of cells into which the material is injected divided by a total number of cells passing through the cell transfer channel, times 100.

8. The method of claim 1, wherein, after the step (c), cell viability is 80% or higher, and wherein the cell viability is defined as a number of viable cells divided by a total number of cells into which the material is injected, times 100.

9. The method of claim 1, wherein:
the first, second, and third channels are radially arranged about the injector,
central portions of the first, second, and third channels are bent at predetermined angles,
the central portions of the first channel and the third channel are connected to each other via the cell transfer channel, and
the central portion of the second channel is connected to the cell transfer channel via the injector.

10. The method of claim 9, wherein the cell transfer channel has a cross-sectional shape that allows a tight contact with the cell therein.

11. The method of claim 9, wherein the cell transfer channel has a cross-sectional size that allows a tight contact with the cell therein.

12. The method of claim 9, wherein the device further comprises a fourth channel through which a material is injected.

13. The method of claim 9, wherein an inner diameter of a central portion of the cell transfer channel is smaller than inner diameters of opposite ends of the cell transfer channel.

14. The method of claim 13, wherein the inner diameter of the central portion of the cell transfer channel is in a range from 2 µm to 200 µm.

15. The method of claim 13, wherein the inner diameters of the opposite ends of the cell transfer channel are in a range from 5 µm to 500 µm.

16. The method of claim 1, wherein the injector has an inner diameter in a range from 50 nm to 5,000 nm.

17. A device for controlling delivery of a material into a cell, the device comprising:
a first channel through which the cell is injected;
a second channel through which the material is injected;
a third channel in which the cell with the material injected therein is harvested;
a cell transfer channel connecting the first channel to the third channel;
a detector configured to detect a presence of the cell; and
an injector configured to receive a signal from the detector and inject the material into the cell.

18. The device of claim 17, wherein:
the first, second, and third channels are radially arranged about the injector, central portions of the first, second, and third channels are bent at predetermined angles,
the central portions of the first channel and the third channel are connected to each other via the cell transfer channel, and
the second channel is connected to the cell transfer channel via the injector.

19. The device of claim 17, further comprising a fourth channel through which a material is injected.
